# EUROPÄISCHE PATENTSCHRIFT

(11) **EP 0 694 295 B1**
(45) Veröffentlichungstag und Bekanntmachung des Hinweises auf die Patenterteilung: **04.08.1999**
(21) Anmeldenummer: 95105497.2
(22) Anmeldetag: 12.04.1995
(51) Int. Cl.: A61F 2/76

(54) **Rohradapter**
Tube adapter
Adaptateur pour tube

(30) Priorität: 30.05.1994 DE 9408804 U
(43) Veröffentlichungstag der Anmeldung: 31.01.1996
(73) Patentinhaber: Otto Bock Orthopädische Industrie Besitz- und Verwaltungs-Kommanditgesellschaft, 37115 Duderstadt (DE)
(72) Erfinder: Hiemisch, Christian, D 37115 Duderstadt (DE); Holzapfel, Markus, D 37520 Osterode (DE)
(74) Vertreter: Gramm, Werner, Prof. Dipl.-Ing.

(56) Entgegenhaltungen:
- EP-A- 0 095 567
- WO-A-94/04102
- CH-A- 499 982
- DE-A- 1 566 404
- DE-U- 9 408 804
- FR-A- 779 037
- FR-A- 2 410 998
- US-A- 4 676 800

## Beschreibung

Die Erfindung betrifft einen Rohradapter zur klemmenden Verbindung mit dem Ende eines Rohrteiles einer Modular-Prothese, bestehend aus einer axial geschlitzten, über eine Spannschraube oder dergleichen umfangsverspannbare, zur Aufnahme des Rohrendes bestimmten Rohrmuffe, an deren eines Ende sich ein ringförmiger Kupplungsabschnitt anschließt, der auf seinem Umfang vorzugsweise vier gleich voneinander beabstandete Gewindebohrungen zur Aufnahme je einer Kupplungs- und Justierschraube und stirnseitig eine ringförmige Kugelpfanne aufweist zur Anlage an einer Kugelkalotte eines Justierkernes, der in Kupplungsstellung mit einem als mehrflächige Pyramide ausgebildeten Ansatz bis in den Klemmbereich der genannten Schrauben ragt.

Eine derartige Ausführungsform läßt sich der US-Patentschrift 3,659,294 (CH-A-499 982) entnehmen. Insbesondere die Figuren 3 und 4 lassen erkennen, daß der Ansatz des Justierkerns als vierflächige Pyramide ausgebildet ist, die auf ihrer Spitze stehend zentrisch zur Kugelkalotte angebracht ist. In der Kupplungsstellung ragt dieser Ansatz durch die ringförmige Kugelpfanne des Adapterteils hindurch bis in den Bereich der Klemmschrauben, die je eine der Pyramidenflächen beaufschlagen, angenähert senkrecht auf dieser Pyramidenfläche stehen und daher mit ihren Schraubachsen unter einem Spitzen Winkel zur Rohrquerschnittsebene angeordnet sind.

Die Klemmschrauben übernehmen die Kupplung zwischen dem Adapterteil und dem Justierkern. Zugleich aber dienen die Klemmschrauben zur Justierung der Mittelachse des Justierkerns gegenüber der Rohrachse des Rohrteils. Durch Lösen der einen Klemmschraube und durch Nachspannen der ihr gegenüberliegenden Klemmschraube ist eine Verschwenkung des Justierkerns gegenüber dem Rohradapter in einer bestimmten Ebene möglich. Durch entsprechende Verstellung der anderen beiden sich gegenüberliegenden Klemmschrauben läßt sich eine Verschwenkung in einer zweiten, senkrecht auf der ersten Ebene stehenden Ebene realisieren, wobei jeweils der Justierkern mit seiner Kugelkalotte in der ringförmigen Kugelpfanne des Adapterteils gleitet.

Der Rohradapter ist üblicherweise aus Stahl oder Titan gefertigt, während das Rohrteil aus Aluminium besteht. Bei der vorbekannten Ausführungsform ist das Rohrteil mit seinen beiden Enden jeweils in einen Rohradapter eingeschoben, wobei die Verbindung als Preßsitz oder auch als Klemmverbindung ausgebildet sein kann. Im letzteren Fall ist der Rohradapter mit einer Klemmschelle versehen, die über eine Schraube angezogen werden kann.

Die WO-A-9404102 offenbart eine Modular-Unterschenkelprothese, deren aus Kunststoff bestehendes Rohrteil an seinen beiden Enden jeweils in einem Rohradapter eingespannt ist. Dieser ist becherförmig gestaltet, längs geschlitzt und weist einen sich zum abgerundeten Becherboden hin konisch verjüngenden Innenraum auf, in den eine Kunststoff-Buchse eingeschoben ist. Diese ist mit einem zu ihrer Rotationsachse schräg verlaufenden Bohrung versehen, in die das jeweils eine Ende des Rohrteils mit leichtem Überstand eingeschoben ist. Zum Festspannen des Rohrendes wird auf den freien Becherrand eine Druckmutter aufgeschraubt, die mit einem Ringbund, das aus dem Becher herausrangende freie Ende der genannten Buchse übergreift. Durch Anziehen der Ringmutter wird die ebenfalls konisch gestaltete Buchse in axialer Richtung in den Becher hineingedrückt, der seinerseits beim zunehmenden Aufschrauben der Ringmutter umfangsverspannt wird. Die Klemmung jedes Rohrendes erfolgt ausschließlich über die Buchse; das aus der Buchse herausragende freie Rohrende stützt sich auf dem abgerundeten Becherboden ab. Die axiale Länge der Kunststoff-Buchse entspricht der für die Klemmung wirksamen Axiallänge des Bechers.

Der Erfindung liegt die Aufgabe zugrunde, die Verbindung zwischen Rohradapter und Rohrteil zu verbessern.

Diese Aufgabe wird erfindungsgemäß dadurch gelöst, daß nur der dem Kupplungsabschnitt gegenüberliegende freie Endabschnitt der Rohrmuffe auf seiner inneren Ringfläche mit einer Kunststoff-Klemmanschette bestückt ist.

Diese Erfindung beruht auf folgenden Erkenntnissen: Durch den Klemmvorgang wird das Rohrteil gerade an der Stelle beaufschlagt, an der auch die durch die Prothesenbelastung aufgebrachten Spannungen am höchsten sind. Ferner wurde festgestellt, daß bei den vorbekannten Ausführungsformen jeder Lastwechsel zu einer Relativbewegung zwischen Rohrmuffe und Rohrteil führt, wodurch das Aluminiumrohr beschädigt wird.

Demgegenüber erfolgt erfindungsgemäß die höchste Klemmung nicht mehr unmittelbar am Rohrende sondern in einem Abstand hiervon, so daß der zwischen der Kunststoff-Klemmanschette und dem Kupplungsabschnitt liegende Rohrabschnitt elastischen Verformungen unterworfen werden kann. Im Bereich der höchsten Relativbewegung zwischen Rohrmuffe und Rohrende wird ein metallischer Kontakt zwischen diesen beiden Teilen durch die Kunststoff-Klemmanschette unterbunden. Dynamische Biegeversuche haben ergeben, daß sich hierdurch die Lebensdauer der genannten Bauteile erheblich verlängert.

In der Zeichnung ist eine als Beispiel dienende Ausführungsform der Erfindung dargestellt. Es zeigen:

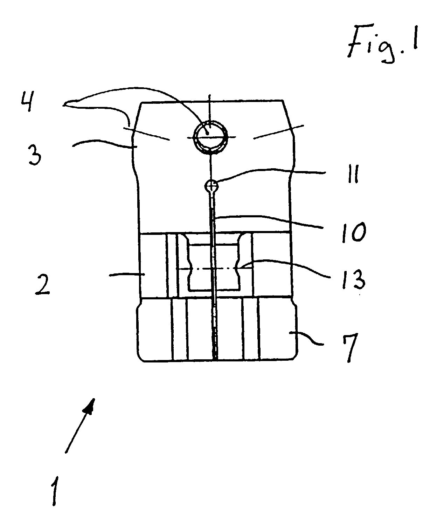

- **Figur 1** -: einen Rohradapter in Seitenansicht und
- **Figur 2** -: in vergrößertem Maßstab einen Längsschnitt durch den Rohradapter gemäß Figur 1 mit einem eingesteckten Rohrteil.

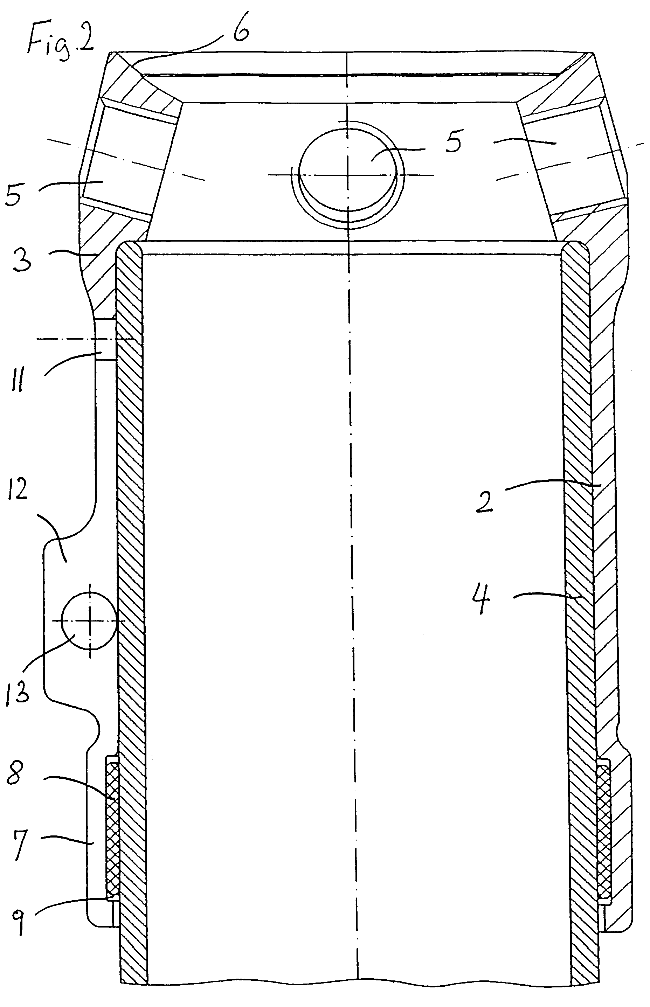

Dargestellt ist ein Rohradapter 1, der aus einer Rohrmuffe 2 und aus einem sich an deren eines Ende anschließenden ringförmigen Kupplungsabschnitt 3 besteht. Figur 2 läßt erkennen, daß die Rohrmuffe 2 zur Aufnahme eines eingesteckten Rohrendes 4 dient.

Der Kupplungsabschnitt 3 weist auf seinem Umfang vier gleich voneinander beabstandete Gewindebohrungen 5 zur Aufnahme je einer in der Zeichnung nicht näher dargestellten Kupplungs- und Justierschraube (Klemmschraube) und stirnseitig eine ringförmige Kugelpfanne 6 auf zur Anlage an einer in der Zeichnung nicht näher dargestellten Kugelkalotte eines Justierkernes, der in Kupplungsstellung mit einem als mehrflächige Pyramide ausgebildeten Ansatz bis in den Klemmbereich der Klemmschrauben ragt.

Die Rohrmuffe 2 ist auf ihrem dem Kupplungsabschnitt 3 gegenüberliegenden Ende mit einem Endabschnitt 7 versehen, der eine Kunststoff-Klemmanschette 8 umschließt, die in eine Ringnut 9 des Endabschnitts 7 eingebettet ist. Die Kunststoff-Klemmanschette 8 kann in die Ringnut 9 lose eingelegt sein. Die Dimensionierung von der Kunststoff-Klemmanschette 8 bzw. der Ringnut 9 wird so gewählt, daß die Klemmung über die gesamte axiale Länge der Rohrmuffe 2, insbesondere aber auch über die axiale Länge der Kunststoff-Klemmanschette 8 erfolgt.

Die Rohrmuffe 2 weist einen Axialschlitz 10 auf, der in eine Entlastungsbohrung 11 übergeht. Beidseitig des Axialschlitzes 10 ist die Rohrmuffe 2 mit je einer Fahne 12 versehen, die je eine miteinander fluchtende Bohrung 13 zur Aufnahme einer nicht näher dargestellten Spannschraube aufweisen.

## Patentansprüche

1. Rohradapter zur klemmenden Verbindung mit dem Ende eines Rohrteiles einer Modular-Prothese, bestehend aus einer axial geschlitzten, über eine Spannschraube oder dergleichen umfangsverspannbare, zur Aufnahme des Rohrendes (4) bestimmten Rohrmuffe (2), an deren eines Ende sich ein ringförmiger Kupplungsabschnitt (3) anschließt, der auf seinem Umfang vorzugsweise vier gleich voneinander beabstandete Gewindebohrungen (5) zur Aufnahme je einer Kupplungs- und Justierschraube und stirnseitig eine ringförmige Kugelpfanne (6) aufweist zur Anlage an einer Kugelkalotte eines Justierkernes, der in Kupplungsstellung mit einem als mehrflächige Pyramide ausgebildeten Ansatz bis in den Klemmbereich der genannten Schrauben ragt, **dadurch gekennzeichnet**, daß nur der dem Kupplungsabschnitt (3) gegenüberliegende freie Endabschnitt (7) der Rohrmuffe (2) auf seiner inneren Ringfläche mit einer Kunststoff-Klemmanschette (8) bestückt ist.

2. Rohradapter nach Anspruch 1, **dadurch gekennzeichnet**, daß die Kunststoff-Klemmanschette (8) als separater Ring ausgebildet ist, der in eine entsprechende Ringnut (9) im Endabschnitt (7) der Rohrmuffe (2) eingebettet ist.

3. Rohradapter nach Anspruch 1, **dadurch gekennzeichnet**, daß die Kunststoff-Klemmanschette (8) als Innenbeschichtung der Rohrmuffe (2) ausgebildet ist.

4. Rohradapter nach Anspruch 1, 2 oder 3, **dadurch gekennzeichnet**, daß die axiale Länge der Kunststoff-Klemmanschette (8) etwa ein Fünftel der Axiallänge der Rohrmuffe (2) entspricht.

## Claims

1. Tube adapter for clamping connection to the end of a tubular part of a modular prosthesis, comprising an axially slotted connecting sleeve (2) which is intended to receive the tube end (4), is circumferentially securable by means of a tightening screw or the like and is adjoined at its one end by an annular coupling portion (3), which comprises at its periphery preferably four uniformly spaced-apart threaded bores (5) for receiving in each case a coupling and adjusting screw and at its end face an annular ball socket (6) for application against a ball joint of an adjusting core which, in a coupling position, protrudes with a projection in the form of a multi-faced pyramid as far as into the clamping region of said screws, **characterized in** that only the free end portion (7) of the connecting sleeve (2) remote from the coupling portion (3) is provided at its inner annular surface with a plastic clamping collar (8).

2. Tube adapter according to claim 1, **characterized in** that the plastic clamping collar (8) takes the form of a separate ring which is embedded in a corresponding annular groove (9) in the end portion (7) of the connecting sleeve (2).

3. Tube adapter according to claim 1, **characterized in** that the plastic clamping collar (8) takes the form of an inner coating of the connecting sleeve (2).

4. Tube adapter according to claim 1, 2 or 3, **characterized in** that the axial length of the plastic clamping collar (8) corresponds to approximately a fifth of the axial length of the connecting sleeve (2).

## Revendications

1. Adaptateur pour tube, destiné à être relié par serrage avec l'extrémité d'une partie tubulaire d'une prothèse modulaire, se composant d'un manchon tubulaire (2) fendu axialement destiné recevoir l'extrémité du tube (4) et pouvant être soumis à un serrage périphérique au moyen d'une vis de serrage ou analogue, tandis qu'un tronçon d'accouplement annulaire (3) raccordé à une première extrémité du manchon tubulaire présente d'une part, sur sa périphérie, de préférence quatre alésages filetés (5) également écartés entre-eux pour recevoir chacun une vis d'accouplement et d'ajustement et d'autre part, frontalement, une cuvette annulaire de rotule (6) destinée à s'appuyer sur une calotte sphérique d'une noix d'ajustement, qui en position d'accouplement s'étend par un appendice réalisé sous la forme d'une pyramide multi-faces jusque dans la zone de serrage des vis précitées, caractérisé en ce que seul le tronçon d'extrémité libre (7) du manchon tubulaire (2), opposé au tronçon d'accouplement (3), est équipé d'une manchette de serrage en matière synthétique (8) sur sa face annulaire intérieure.

2. Adaptateur pour tube selon la revendication 1, caractérisé en ce que la manchette de serrage en matière synthétique (8) est réalisée sous la forme d'une bague séparée, qui est logée dans une rainure annulaire correspondante (9) dans le tronçon d'extrémité (7) du manchon tubulaire (2).

3. Adaptateur pour tube selon la revendication 1, caractérisé en ce que la manchette de serrage en matière synthétique (8) est réalisée sous la forme d'un revêtement intérieur du manchon tubulaire (2).

4. Adaptateur pour tube selon la revendication 1, 2 ou 3, caractérisé en ce que la longueur axiale de la manchette de serrage en matière synthétique (8) correspond à environ un cinquième de la longueur axiale du manchon tubulaire (2).
